Europäisches Patentamt

European Patent Office

Office européen des brevets

(11) Publication number:

**0 150 288**
A2

## (12) EUROPEAN PATENT APPLICATION

(21) Application number: 84113631.0

(22) Date of filing: 12.11.84

(51) Int. Cl.⁴: **C 03 C 11/00**, C 03 C 17/00, C 12 N 11/00, C 04 B 14/00

(30) Priority: 16.01.84 US 571375

(43) Date of publication of application: 07.08.85
Bulletin 85/32

(84) Designated Contracting States: CH DE FR GB IT LI SE

(71) Applicant: KMS Fusion, Inc., 3941 Research Park Drive, Ann Arbor Michigan 48106 (US)

(72) Inventor: Anderson, Paul Richard, 326 Rawson, Dundee Michigan 48131 (US)
Inventor: Wuttke, Gilbert Henry, 339 Willis Road, Saline Michigan 48176 (US)

(74) Representative: Wehnert, Werner et al, Patentanwälte Dipl.-Ing. Hauck, Dipl.-Phys. Schmitz, Dipl.-Ing. Graalfs, Dipl.-Ing. Wehnert, Dipl.-Phys. Carstens, Dr.-Ing. Döring Mozartstrasse 23, D-8000 München 2 (DE)

(54) Hollow microspheres with wall through-openings.

㊻ Hollow microspheres or shells having wall through-openings, and methods for manufacture thereof, which find application as microcarriers for anchorage-dependent cell cultivation and as fillers for thermal insulation purposes. Hollow glass or polymeric shells having removal-susceptible wall zones carried by a wall matrix are chemically treated to remove the susceptible zones and thereby leave the matrix wall with through-openings. The shells may be vacuum coated to obtain a discrete reflective surface layer which seals an evacuated interior, or, in the case of glass, the shells may be vacuum sintered to close the through-openings, and then coated to obtain the reflective surface layer.

STEP I — STARTING MATERIAL

STEP 2 — GLASS SHELL FABRICATION

STEP 3 — HEAT TREAT FOR PHASE SEPARATION

STEP 4 — SELECTIVE PHASE REMOVAL

GLASS SHELL WITH WALL THROUGH-OPENINGS

RAC/RCC/srs

HOLLOW MICROSPHERES WITH
WALL THROUGH-OPENINGS

The present invention is directed to hollow microspheres, and to methods for manufacture thereof.

Background of the Invention

Hollow micro-sized shells, also known as microspheres
or microballoons, are finding ever-increasing utility in
the arts. One recent application of microsphere technology is in the field anchorage-dependent cell cultivation
wherein glass-surfaced microspheres are employed as cell
microcarriers. United States patent application Serial
Nos. 332,377 and 354,345 respectively filed December 21,
1981 and March 3, 1982, both assigned to the assignee
hereof, provide microspheres having spherically continuous
glass surfaces for cell attachment and having densities
tailored closely to match that of the culture medium.
The latter feature presents a highly desirable improvement
by permitting the microcarriers to remain suspended in the
culture medium with minimal agitation. The technology

-2-

disclosed in these applications has been successfully implemented, but is more expensive than desired for high-volume commercial applications. One important aspect of the present invention contemplates provision of hollow microspheres for microcarrier applications which embody the desirable features of glass for cell attachment, which are readily suspendable in a cell culture medium with little or no agitation, and which are more economical to implement than technology heretofore proposed.

Another application of microsphere technology which is enjoying increased interest is in the field of thermal insulation. United States Patent No. 4,303,732, for example, discloses a thermal barrier wherein microspheres are employed as a filler. The microspheres are of glass construction, and may be made to enclose a high vacuum and a thin metal coating deposited on the inner surface of the shell wall. Manufacture is accomplished by using a blowing gas of metallic vapor to expand a molten glass film, which gas thereafter condenses on the shell wall as the shell cools, leaving a high vacuum encapsulated within the shell. In addition to being somewhat expensive to implement, disposition of the heat-reflective coating on the shell wall inside surface permits the shell itself to be heated. A second important aspect of the present

0150288

invention contemplates provision of an improved method for manufacturing microspheres for thermal insulation applications, and to the resulting product, wherein the shell interior is evacuated and the heat-reflective metallic coating is disposed on the outer shell surface.

## Objects and Summary of the Invention

In addition to the foregoing, another important object of the invention is to provide an improved method for manufacturing hollow microspheres, including all-glass microspheres, having an evacuated interior.

In accordance with the present invention, hollow spherical shells or microspheres having wall through-openings are provided by first fabricating shells having spherically continuous (i.e. closed) walls in which discrete wall zones having physical and/or chemical properties rendering them susceptible to removal are carried in a matrix formed by the remainder of the wall having different physical and/or chemical properties. The shells are then treated selectively to remove the susceptible zones while leaving the surrounding matrix intact. The resulting openings extend through the shell wall from interior to exterior. In one implementation of the invention, starter shells are of silicate glass composition which is either phase-separated in the manufactur-

-4-

ing process or in a subsequent heat-treating operation to form silica-deficient zones surrounded by a silica-rich matrix. The silica-deficient zones are removed in a leaching operation, leaving a silica-rich shell having wall through-openings. In microcarrier applications for cell cultivation, the wall through-openings permit the culture medium to enter the shell interior, so that the shell itself exhibits substantially neutral buoyancy (neither floats nor sinks), and thus remains suspended for substantial periods of time with minimal agitation.

In another implementation of the invention, starter shells of polymeric construction are selectively irradiated to create damage tracks within the otherwise cross-linked wall microstructure. These damaged zones are removed to leave a polymeric shell having through-the-wall openings.

For insulation applications, the hollow glass or polymeric shells may be vacuum coated to obtain a discrete surface layer of reflective metallic construction and an evacuated interior sealed by the surface layer. Alternatively, the glass shells may first be subjected to a vacuum sintering operation to close the wall through-openings and thereby seal the evacuated shell interior. The closed and evacuated shell may then be coated to obtain the exterior metallic layer.

Brief Description of the Drawings

The invention, together with additional objects, features and advantages thereof, will be best understood from the following description, the appended claims and the accompanying drawings in which:

FIG. 1 is a process flow chart for manufacturing glass shells having wall through-openings in accordance with one aspect of the invention;

FIG. 2 is a corresponding flow chart for manufacture of polymeric shells;

FIGS. 3-4 are SEM micrographs at 10,000X magnification which illustrate phase separation of glass shells;

FIGS. 5-9 are SEM micrographs which illustrate the wall through-openings in various glass shells in accordance with the invention, FIG. 5 being at 5000X magnification and FIGS. 6-9 being at 10,000X magnification; and

FIG. 10 is a process flow chart for obtaining coated evacuated shells in accordance with the invention.

-6-

Detailed Description of Preferred Embodiments

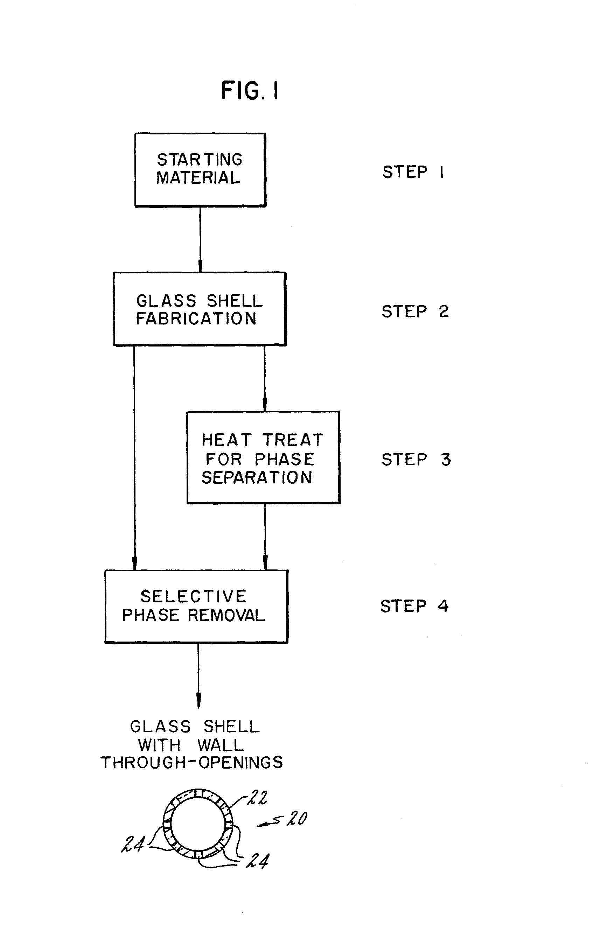

FIG. 1 is a process flow chart for obtaining silicate glass shells with through-the-wall openings in accordance with the invention. There are many techniques for fabrication of glass microspheres known in the art. The physical state of the starting material depends upon the technique employed, and may comprise a dried and crushed metal organic gel, a liquid metal organic gel, glass frit, or an aqueous collodial suspension or sol. Likewise, the manner of shell fabrication depends upon the starting material employed, and may include dropping gel or frit particles through a tower furnace, spray-atomization of a liquid gel or sol into a furnace, or flame spraying of gel or frit particles. Standard techniques for glass microsphere fabrication are variously illustrated, for example, in United States Patent Nos. 3,365,315, 4,017,290, 4,021,253, 4,336,338 and 4,340,407. Thus, in general, Steps 1 and 2 in FIG. 1 are conventional, and result in an intermediate shell product having a spherically continuous (i.e. closed) wall structure and a hollow interior.

It is important in implementation of the process of FIG. 1 in accordance with the present invention that the composition of the starting material be selected in conjunction with the method of shell fabrication so that the resulting intermediate shell product (of Step 2) be of multicomponent glass composition wherein silica is the major constituent, and that the shell will be either phase separated or capable of phase separation into silica-deficient zones carried by and separated from each other by a silica-rich matrix.  The art of phase separation in silicate glasses is not well understood.  Doremus, Glass Science, John Wiley & Sons (1973), especially Chapter 4 entitled "Phase Separation", provides a background discussion of this art.  Most desirably, in terms of economy, the shells would undergo sufficient phase separation during the fabrication process.  However, where such does not occur, or for better control of the degree of phase separation, the shells may be subjected to a separate heat treat operation (Step 3) to promote phase separation.  Such heat treatment is accomplished at a temperature less than the shell softening temperature, and for a time empirically determined for each glass composition and fabrication method to yield the desired amount of phase separation.

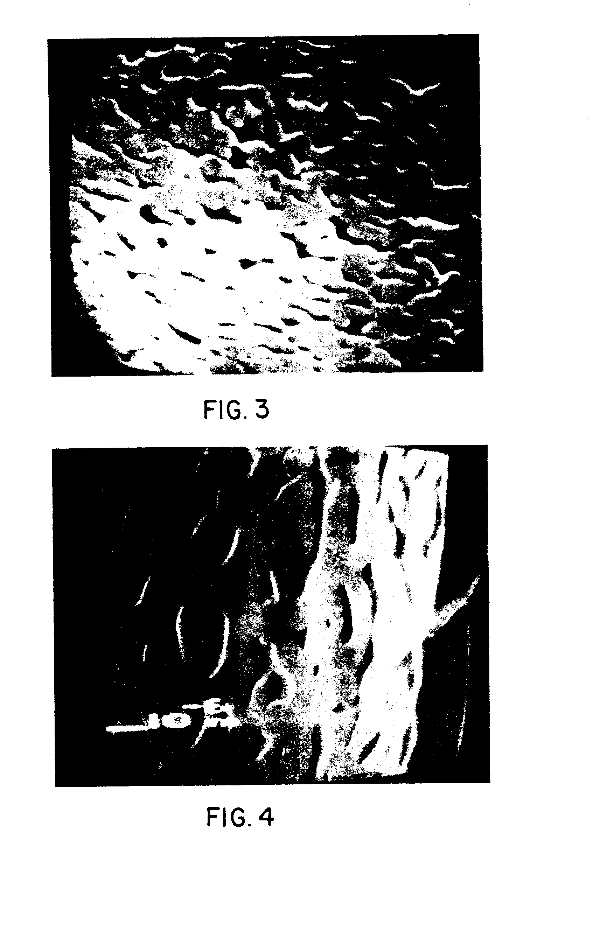

In one working implementation of the process of FIG. 1, a metal organic gel was formed to contain Na, K, Ca and Si as the glass-forming components.  The gel was dried and crushed, and the resulting gel particles were dropped through a tower furnace containing a substantial amount of water vapor. The presence of water vapor, long furnace residence time ( ~ 10 to 30 seconds) and high furnace temperature (1500°C) promote substantial volatilization of the Na and K oxides, resulting in glass shells consisting essentially of about 10% CaO, less than 2% $Na_2O$ and $K_2O$, and the remainder $SiO_2$. (All percentages given in the present application are in terms of oxide-equivalent mole percent.) The resulting shells, having spherically continuous walls, were phase separated as a result of the manufacturing process. FIGS. 3 and 4 are micrographs of the interior of two resulting shells, and illustrate phase separation into calcia-rich (silica deficient) zones separated from each other and carried by a surrounding silica-rich matrix. In this connection, it will be appreciated that the amount of phase separation and the size of the silica-deficient zones will vary with glass composition and fabrication and/or heat-treat parameters, which must be determined empirically for each application.

Glasses of the so-called VYCOR (trademark) composition, consisting of $Na_2O$, $B_2O_3$ and $SiO_2$, may be readily phase separated by heat treatment into a silica-rich phase and a $Na_2O-B_2O_3$-rich (silica-deficient) phase. The same is true of the binary glass composition consisting essentially of about 10% soda and the remainder silica. Phase separability of other glass compositions, and the required parameters, must be determined empirically. In general, it is believed that glass compositions which exhibit a "two liquid" region within the high-silica portion of the associated phase diagram may be employed, and $CaO-SiO_2$ and $Na_2O-CaO-SiO_2$ are examples of such compositions.

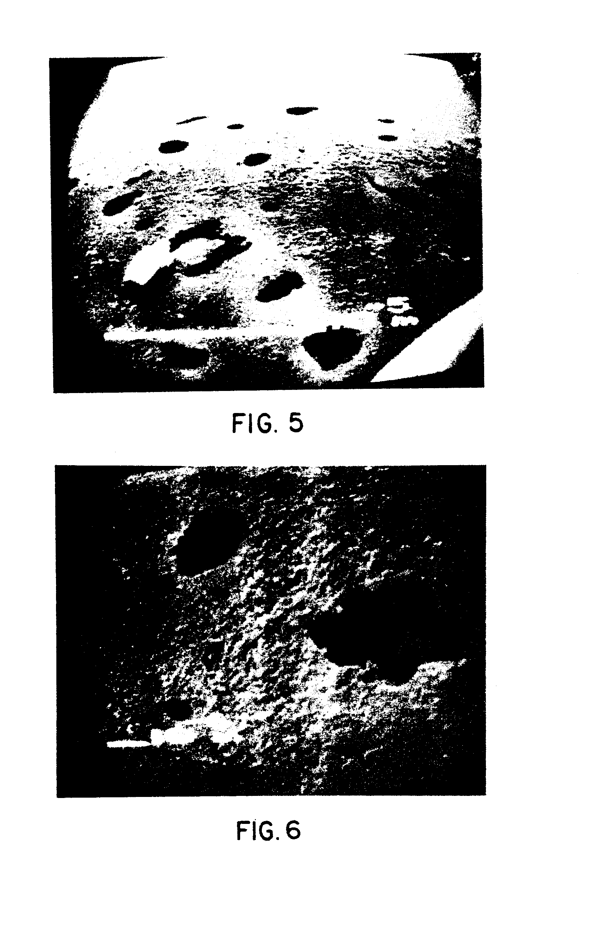

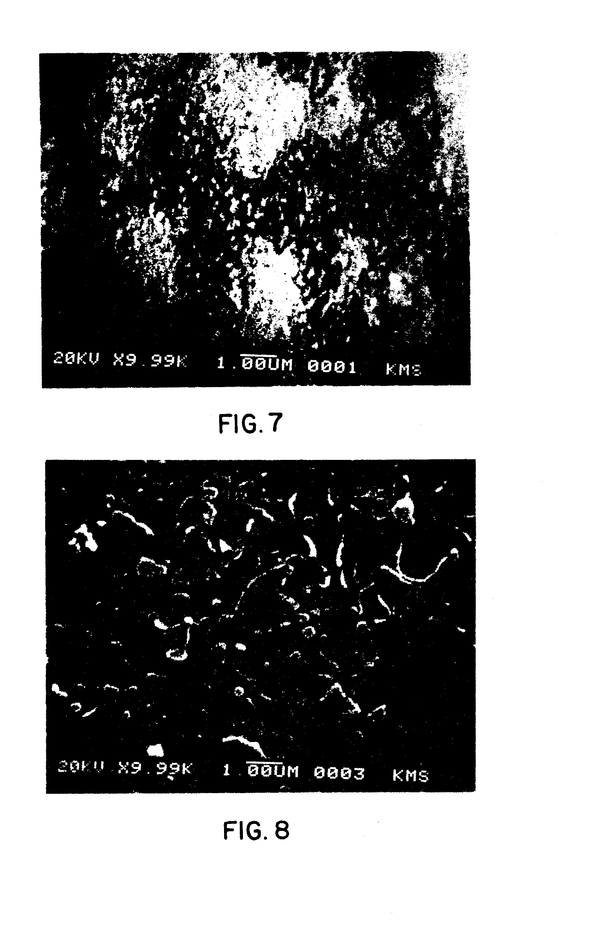

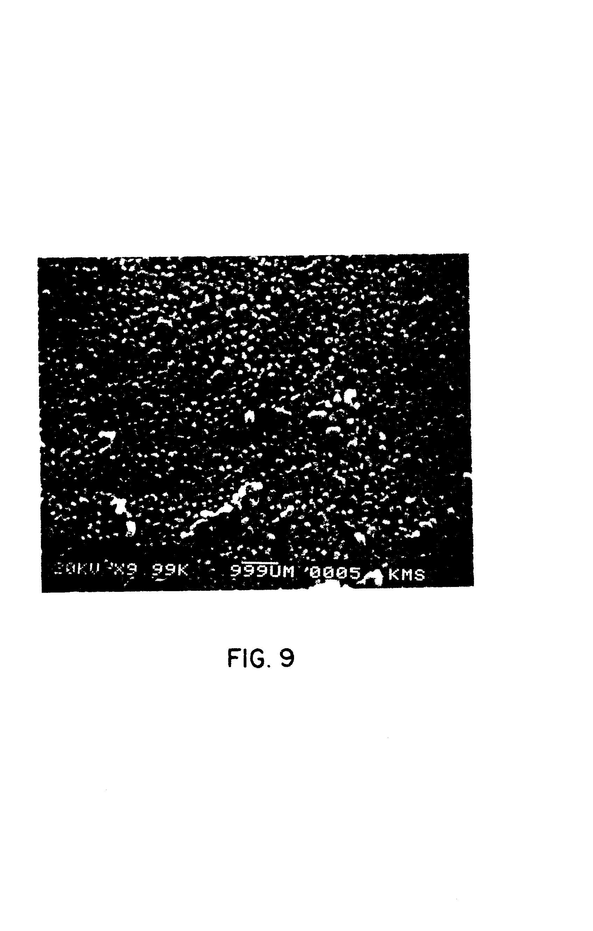

Returning to FIG. 1, the phase-separated shells are next subjected to a treatment (Step 4) for selective removal of the more soluble silica-deficient phase. The silica-deficient phase may be readily leached by various dilute mineral acids. The product which remains is a hollow spherical shell 20 consisting of the silica-rich matrix wall 22 and a multiplicity of wall through-openings 24, where the silica-deficient zones had been dissolved, extending through the matrix wall from exterior to interior. FIGS. 5-9 illustrate such shell products constructed in accordance with the present invention. FIGS. 5 and 6 are exterior micrographs of the same shell, with FIG. 6 being at twice the magnification of FIG. 5. The through-openings are quite large and are readily visible as such. (Note that all FIGS. 3-9 have the appropriate scale indicated therein.) FIGS. 7-9 are exterior micrographs of three respectively different shells from a shell batch different from that of FIGS. 5-6. The through-openings are of progressively decreasing size in FIGS. 7-9, illustrating
differences obtained due to differing fabrication parameters and amounts of phase separation.

In use as microcarriers for anchorage-dependent cell
cultivation, the glass shells resulting from the process
of FIG. 1 exhibit substantially neutral buoyancy in the
culture medium. The medium permeates the through-openings,
so that only the relatively small mass of the shell matrix
wall affects buoyancy. The shells of the invention remain
in suspension for substantial periods with little or no
agitation and settle slowly to the bottom, which
facilitates removal. For microcarrier applications, a
shell diameter of 50-300 microns and an aspect ratio of
at least 5/1 are suitable.

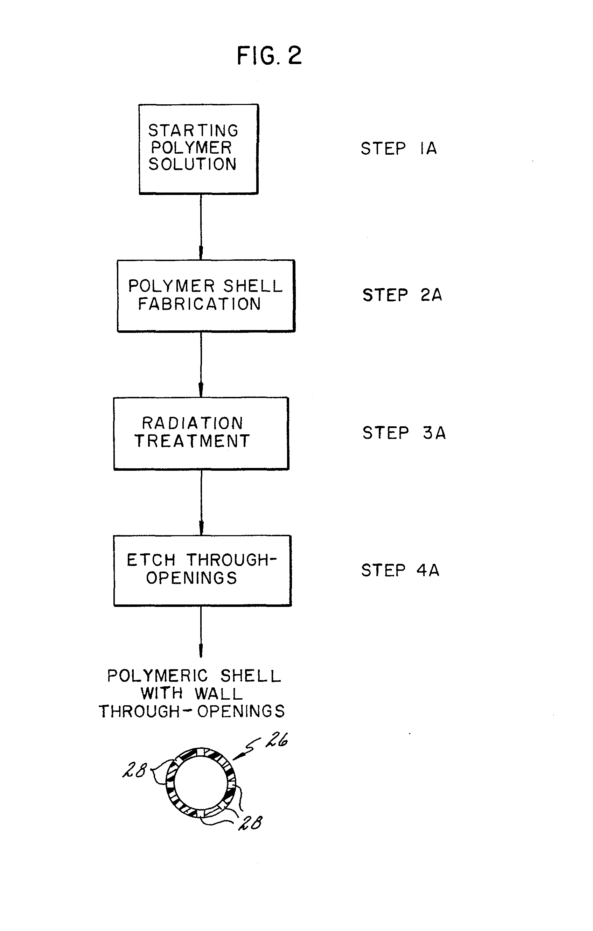

Polymeric shells with wall through-openings may be
constructed following the process of FIG. 2. A suitable
polymer solution is formed (Step 1A) and polymeric shells
are formed (Step 2A) employing conventional technology.
United States Patent Nos. 2,797,201 and 4,405,373 are
illustrative of this art. To implement the process of
FIG. 2 in accordance with the present invention, the
polymer composition must be of a type sensitive to radiation to induce cross-linkage. The polycarbonates and other materials disclosed in United States Patent No. 3,802,972 would be suitable, by way of example. The shells resulting from Step 2A are subjected to selective radiation treatment (Step 3A) to form cross-linked through-zones in the shell wall, which may then be removed (Step 4A) in an etching operation using sodium or potassium hydroxide. The result is a polymeric shell 26 with wall through-openings 28.

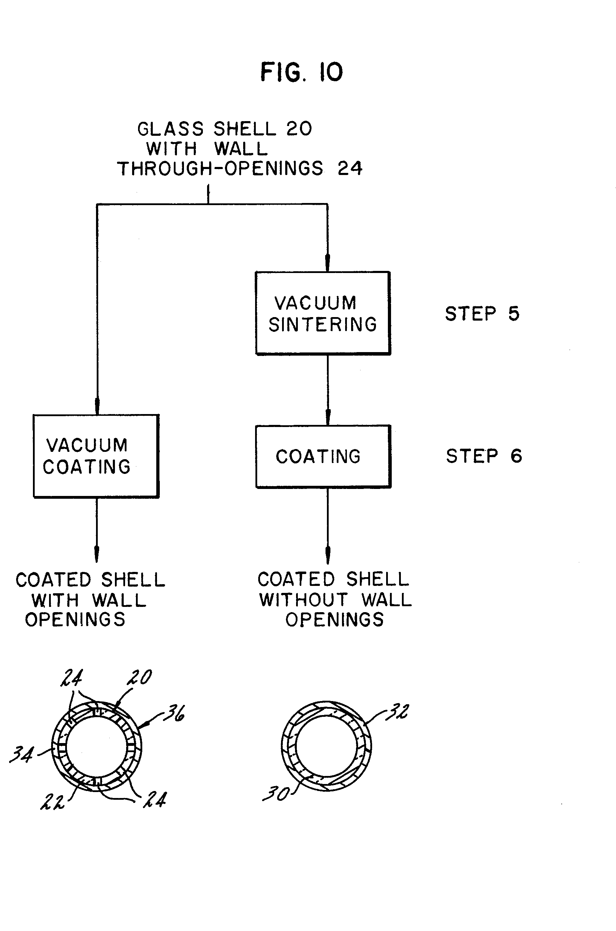

FIG. 10 illustrates further processing the shells of FIG. 1 in accordance with the thermal insulating aspects of the invention. Glass shells 20 with wall through-openings 24 are subjected to a vacuum sintering operation (Step 5) so as to close the wall through-openings and thereby seal the evacuated interior. A sintering temperature in the range of 65% to 90% of the shell melting temperature would be suitable. The resulting evacuated shell 30 is then coated (Step 6) to form a discrete layer 32 on the outer shell surface. Preferably such layer consists of a heat-reflective metallic material on the order of 1000Å thick. Conventional chemical or physical vapor deposition and rf or dc sputtering processes would be suitable.

As an alternative, glass shell 20 may be coated directly in a high vacuum coating operation (Step 6) so as

-12-

to deposit a discrete metallic reflective surface layer 34 thereon. Layer 34 serves both to seal the evacuated interior of the composite shell 36, and to reflect heat from the shell body. Polymeric shells 26 (FIG. 2) may be vacuum coated to obtain a similar product. In this embodiment, a coating of about 1000$\overset{o}{A}$ thickness is desirable. Conventional high vacuum e-beam evaporation and physical vapor deposition processes are suitable. Aluminum would be a suitable metal for either of the layers 32, 34 in FIG. 10.

The invention claimed is:

1. A method of providing a hollow spherical shell having shell wall through-openings comprising the steps of:

(a) providing a hollow spherical shell having a closed spherically continuous wall in which zones extending through said wall and having first properties are separated from each other by a matrix having differing properties, and

(b) treating said shell selectively to remove said zones while leaving said matrix intact so as to provide openings in place of said zones extending through said matrix.

2. The method set forth in claim 1 wherein said step (a) comprises the step of providing a said closed hollow spherical shell of multicomponent silicate glass composition which is phase-separated to possess a silica-deficient phase at said zones and a silica-rich phase as said matrix, and

wherein said step (b) comprises the step of chemically leaching said silica-deficient phase from the surrounding said matrix.

3. The method set forth in claim 2 wherein said step (a) comprises the steps of (a1) providing said shell of substantially homogeneous silicate glass composition, and then (a2) heat treating said shell to obtain said phase separation.

4. The method set forth in claim 2 comprising the additional step of: (c) sintering said shell matrix to provide a hollow spherical shell product having a closed spherically continuous wall and a sealed interior.

5. The method set forth in claim 4 wherein said step (c) comprises the step of vacuum sintering said shell matrix to provide said shell product having an evacuated interior.

6. The method set forth in claim 2 comprising the additional step of: (c) depositing on said shell matrix a distinct continuous surface layer of heat-reflective metallic composition.

7. The method set forth in claim 6 wherein said step (c) comprises the step of vacuum coating said shell matrix to provide a coated matrix product having an evacuated interior.

-15-

8. The method set forth in claim 6 comprising the additional step prior to said step (c) of: (d) sintering said shell matrix to close said through-openings.

9. The method set forth in claim 8 wherein said step (d) comprises the step of vacuum sintering said matrix, and

wherein said step (c) comprises the step of coating the sintered shell matrix to provide a coated product having an evacuated interior.

10. The method set forth in claim 1 wherein said step (a) comprises the steps of:

(a1) providing a said hollow spherical shell of homogeneous radiation-sensitive polymeric composition, and (a2) irradiating said shell selectively to damage said polymeric composition in said zones, and

wherein said step (b) comprises the step of etching the irradiated shell to remove said zones.

-16-

11.   The method set forth in claim 10 comprising the additional step of:   (c) depositing on said shell matrix a distinct continuous surface layer of heat-reflective metallic composition.

12.   The method set forth in claim 11 wherein said step (c) comprises the step of vacuum coating the shell matrix to provide a coated matrix product having an evacuated interior.

13.   A method of fabricating a microcarrier for anchorage-dependent cell cultivation comprising the steps of:

(a) providing a hollow spherical shell having a closed spherically continuous wall of outside diameter in the range of 50 to 300 microns in which discrete zones extend through said wall, have first properties susceptible to chemical attack and are separated from each other by a matrix having differing properties resistant to chemical attack, and

(b) chemically removing said discrete zones to provide a shell product consisting of said matrix having through-openings in place of said zones.

14. The method set forth in claim 13 wherein said shell has a composition selected from the group consisting of: (1) phase-separated silicate glasses having a silica-deficient phase as said zones and a silica-rich phase as said matrix, and (2) radiation-sensitive polymers having radiation-damages tracks as said zones.

15. A method of fabricating a hollow shell for thermal insulation applications comprising the steps of:

(a) providing a hollow shell having a spherical wall with a multiplicity of wall through-openings extending from exterior to interior of said shell,

(b) evacuating the interior of said shell, and

(c) depositing on the external surface of said shell wall a discrete continuous surface layer of heat-reflective metallic composition.

16. The method set forth in claim 15 wherein said steps (b) and (c) are performed simultaneously in a vacuum coating operation, with said surface layer sealing the evacuated interior of said shell.

0150288

-18-

17.  The method set forth in claim 15 wherein said shell is of silicate glass composition, and wherein said step (b) comprises the step of vacuum-sintering said shell so as to close said through-openings and seal the evacuated interior of the sintered wall.

18.  The method set forth in claim 15 wherein said step (a) comprises the steps of:

(a1) providing a said shell of multicomponent silicate glass composition phase-separated to possess a silica-deficient phase surrounded by a silica-rich matrix, and

(a2) chemically leaching said silica-deficient phase from said matrix to leave said through-openings.

19.  The method set forth in claim 15 wherein said step (a) comprises the steps of:

(a1) providing a said shell of polymeric composition having spherically spaced zones of radiation-damaged microstructure surrounded by a polymeric matrix of non-damaged microstructure, and

(a2) chemically leaching said zones from said matrix to leave said through-openings.

-19-

20.  A hollow spherical shell having a spherical wall with an outside diameter in the range of 50 to 300 microns and an aspect ratio of at least 5/1, and a multiplicity of spaced openings extending through said wall from interior to exterior of said shell.

21.  The shell set forth in claim 20 wherein said wall has a composition selected from the group consisting of polymers and silicate glasses.

22.  The shell set forth in claim 21 further comprising a discrete surface layer of reflective metallic construction surrounding said wall and sealing said interior, said interior being evacuated.

23.  A hollow shell for use in thermal insulating applications, said shell comprising a spherical wall of substantially uniform thickness, an evacuated interior, and a discrete surface layer of reflective metallic composition surrounding and carried by said wall.

24.  The hollow shell set forth in claim 23 wherein said wall has a composition selected from the group consisting of silicate glasses and polymers.

25. A hollow spherical shell constructed in accordance with the method set forth in claim 1.

26. A hollow spherical shell constructed in accordance with the method set forth in claim 2.

27. A hollow spherical shell constructed in accordance with the method set forth in claim 10.

28. A microcarrier constructed in accordance with the method set forth in claim 13.

29. A hollow spherical shell constructed in accordance with the method set forth in claim 18.

30. A hollow spherical shell constructed in accordance with the method set forth in claim 19.

## FIG. I

```
┌─────────────┐
│  STARTING   │      STEP 1
│  MATERIAL   │
└─────────────┘
      │
      ▼
┌─────────────┐
│ GLASS SHELL │      STEP 2
│ FABRICATION │
└─────────────┘
      │
      ▼
┌─────────────┐
│ HEAT TREAT  │
│ FOR PHASE   │      STEP 3
│ SEPARATION  │
└─────────────┘
      │
      ▼
┌─────────────┐
│  SELECTIVE  │      STEP 4
│PHASE REMOVAL│
└─────────────┘
      │
      ▼
 GLASS SHELL
  WITH WALL
THROUGH-OPENINGS
```

FIG. 2

| | |
|---|---|
| STARTING POLYMER SOLUTION | STEP IA |

↓

| | |
|---|---|
| POLYMER SHELL FABRICATION | STEP 2A |

↓

| | |
|---|---|
| RADIATION TREATMENT | STEP 3A |

↓

| | |
|---|---|
| ETCH THROUGH-OPENINGS | STEP 4A |

↓

POLYMERIC SHELL
WITH WALL
THROUGH-OPENINGS

FIG. 3

FIG. 4

FIG. 5

FIG. 6

20KV X9.99K   1.00UM 0001   KMS

FIG. 7

20KU X9.99K   1 00UM 0003   KMS

FIG. 8

FIG. 9

## FIG. 10

GLASS SHELL 20
WITH WALL
THROUGH-OPENINGS 24

| | | |
|---|---|---|
| | VACUUM SINTERING | STEP 5 |
| VACUUM COATING | COATING | STEP 6 |

COATED SHELL
WITH WALL
OPENINGS

COATED SHELL
WITHOUT WALL
OPENINGS